(19) Europäisches Patentamt
European Patent Office
Office européen des brevets

(11) Veröffentlichungsnummer: **0 220 613 B1**

(12) **EUROPÄISCHE PATENTSCHRIFT**

(45) Veröffentlichungstag der Patentschrift: **08.05.91**

(51) Int. Cl.$^5$: **A61K 7/13**, C07C 229/54, C07C 309/45

(21) Anmeldenummer: **86114301.4**

(22) Anmeldetag: **16.10.86**

# (54) Haarfärbemittel mit direktziehenden Nitrodiphenylamin-Derivaten.

(30) Priorität: **24.10.85 DE 3537763**

(43) Veröffentlichungstag der Anmeldung:
**06.05.87 Patentblatt 87/19**

(45) Bekanntmachung des Hinweises auf die Patenterteilung:
**08.05.91 Patentblatt 91/19**

(84) Benannte Vertragsstaaten:
**AT BE CH DE FR GB IT LI NL SE**

(56) Entgegenhaltungen:
**DE-A- 1 569 808**
**FR-A- 2 195 424**
**FR-A- 2 360 559**
**US-A- 2 687 431**

**F. RICHTER: "Beilsteins Handbuch der organischen Chemie", vierte Auflage, erstes Ergänzungswerk, dreizehnter und vierzehnter Band, als Ergänzung des dreizehnten und vierzehnten Bandes des Hauptwerkes, Seite 564, Deutschen Chemischen Gesellschaft, Verlag von Julius Springer, Berlin, DE;**

(73) Patentinhaber: **Henkel Kommanditgesellschaft auf Aktien**
**Postfach 1100 Henkelstrasse 67**
**W-4000 Düsseldorf-Holthausen(DE)**

(72) Erfinder: **Rose, David, Dr.**
**Holbeinweg 7**
**W-4010 Hilden(DE)**
Erfinder: **Lieske, Edgar**
**Hunsrückenstrasse 40**
**W-4000 Düsseldorf(DE)**
Erfinder: **Maak, Norbert, Dr.**
**Im Jagdfeld 41 a**
**W-4040 Neuss(DE)**

**EP 0 220 613 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Gegenstand der Erfindung sind Haarfärbemittel mit einem Gehalt an direktziehenden Haarfarbstoffen. Solche Haarfärbemittel enthalten direktziehende Haarfarbstoffe in einem kosmetischen Träger. Oft enthalten solche Haarfärbemittel zur Erzielung bestimmter Nuancen zusätzlich Oxidationsfarbstoffvorprodukte. Als kosmetische Träger für die direktziehenden Haarfarbstoffe und gegebenenfalls Oxidationsfarbstoffvorprodukte dienen Cremes, Emulsionen, Gele, Shampoos, Schaumaerosole oder andere Zubereitungen, die für die Anwendung auf dem Haar geeignet sind.

Für das Färben von Haar spielen neben den Oxidationsfarben, die durch oxidative Kupplung einer oder mehrerer Entwicklerkomponenten untereinander oder mit einer oder mehreren Kupplerkomponenten entstehen, besonders die direktziehenden Haarfarbstoffe eine wichtige Rolle. Die direktziehenden Farbstoffe haben den Vorteil, daß sie ohne Zusatz von Oxidationsmitteln zur Anwendung kommen. Als direktziehende Farbstoffe werden vorwiegend Verbindungen eingesetzt, die zur Gruppe der Nitrobenzolderivate gehören. Sie werden entweder allein oder in Kombination mit anderen direktziehenden Farbstoffen wie Anthrachinonfarbstoffen, Indophenolen, Triphenylmethanfarbstoffen, kationischen Azofarbstoffen oder mit Oxidationsfarbstoffen eingesetzt.

Gute Haarfärbemittel müssen die gewünschten Farbnuancen in ausreichender Intensität ausbilden. Sie müssen ein gutes Aufziehvermögen auf menschlichem Haar besitzen, ohne die Kopfhaut zu stark anzufärben. Die damit erzeugten Färbungen müssen eine hohe Stabilität gegen Licht, Wärme, Schweiß, Haarwaschmittel und die bei der Dauerwellung des Haares verwendeten Chemikalien aufweisen. Sie sollen schließlich in toxikologischer und dermatologischer Hinsicht unbedenklich sein.

Unter den direktziehenden Nitrobenzolderivaten spielen die Nitroaniline und deren Derivate eine besondere Rolle, da einige dieser Farbstoffe intensive Färbungen von guter Lichtechtheit ausbilden. Ein Nachteil der bekannten direktziehenden Nitroanilin-Farbstoffe ist jedoch einerseits eine begrenzte Wasserlöslichkeit, die zu Problemen bei der Formulierung der Haarfärbemittel führt, andererseits die unbefriedigende Waschechtheit, d. h. daß die Haarfärbungen nach mehrmaligem Haarwaschen stark ausbluten. Direktziehende Farbstoffe sollen darüber hinaus eine gute Verträglichkeit mit anderen Farbstoffen, z. B. mit Oxidationsfarbstoffvorprodukten und mit den in Oxidationshaarfärbemitteln üblichen Komponenten aufweisen, da man zur Nuancenabwandlung gerne direktziehende Farbstoffe und Oxidationsfarbstoffe kombiniert. Daher ist eine gute Stabilität gegen Reduktionsmittel und gegen Oxidationsmittel erforderlich. Aus DE-A-1 569 808 waren Nitro-p-phenylendiamin-derivate als Farbstoffe für die Haaranfärbung bekannt.

Es wurde gefunden, daß Haarfärbemittel, die als direktziehende Haarfarbstoffe Verbindungen der allgemeinen Formel I in der eine der Gruppen $R^1$ bis $R^4$ eine $-SO_3H$ oder eine -COOH-Gruppe ist und die drei anderen unabhängig voneinander Wasserstoff oder Alkylgruppen mit 1 bis 4 C-Atomen sind oder eine der anderen eine Alkoxygruppe mit 1 bis 4 C-Atomen oder Chlor und zwei Wasserstoff oder Alkylgruppen mit 1 bis 4 C-Atomen sind, und deren wasserlösliche Salze enthalten sind, die gestellten Anforderungen in hohem Maße erfüllen.

Die Farbstoffe der allgemeinen Formel I erzeugen auf dem Haar gelbe bis orangerote Farbnuancen von hoher Intensität, Licht-und Waschechtheit. Sie besitzen darüber hinaus im Vergleich zu bekannten Nitroanilinfarbstoffen eine bessere Löslichkeit im wäßrig-alkalischen Medium. In dermatologischer und toxikologischer Hinsicht sind die Verbindungen der Formel I unschädlich und daher für die Anwendung in Haarfärbemitteln besonders geeignet.

Wegen ihrer leichten technischen Zugänglichkeit enthalten die erfindungsgemäßen Haarfärbemittel bevorzugt solche Verbindungen der Formel I, in der eine der Gruppen $R^1$ bis $R^4$ eine $-SO_3H$ oder eine -COOH-Gruppe ist und die anderen Wasserstoff sind oder eine der anderen eine Methylgruppe, eine Methoxygruppe oder Chlor ist und zwei Wasserstoff sind.

Verbindungen der allgemeinen Formel I, in der $R^2$ oder $R^3$ eine -COOH-Gruppe und $R^1$ und $R^4$ Wasserstoff sind, sind literaturbekannt.

Neu und daher ein weiterer Gegenstand der Erfindung sind Verbindungen der Formel I, in der eine der Gruppen $R^1$ bis $R^4$ eine $-SO_3H$-Gruppe ist und die anderen Wasserstoff sind oder eine der anderen eine Methylgruppe, eine Methoxygruppe oder Chlor ist und zwei Wasserstoff sind und deren wasserlösliche Salze. Neu und ein weiterer Gegenstand der Erfindung sind auch Verbindungen der Formel I, in der eine der Gruppen $R^1$ bis $R^4$ eine -COOH-Gruppe, eine zweite eine Methylgruppe, eine Methoxygruppe oder Chlor ist und zwei Wasserstoff sind und deren wasserlösliche Salze.

Die Herstellung der Verbindungen der allgemeinen Formel I erfolgt allgemein durch Umsetzung von 2,4-Dinitrofluorbenzol mit einem Anilinderivat der Formel II

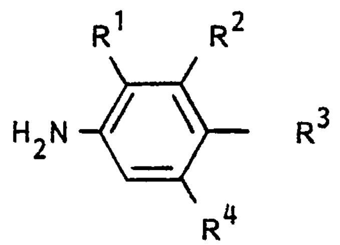

(II)

unter Abspaltung von HF in Gegenwart einer Base zu dem entsprechenden Dinitro-diphenylaminderivat der Formel III

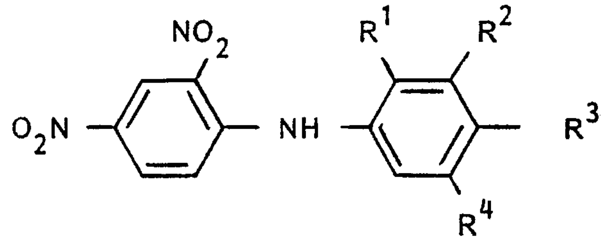

(III)

und Reduktion dieses Dinitro-diphenylaminderivates zum 2-Amino-4-nitro-diphenylamin-Derivat der Formel I. Dabei haben in Formel II und III die Gruppen $R^1$ bis $R^4$ die für Formel I angegebene Bedeutung. Die Reduktion des 2,4-Dinitro-diphenylaminderivats läßt sich besonders gut mit einer Mischung aus Natriumsulfid und Schwefel in wäßrig-alkoholischer Lösung durchführen.

Unter den wasserlöslichen Salzen sind in erster Linie die Salze starker Basen wie etwa die Alkalisalze, z. B. das Natrium- oder Kaliumsalz oder Ammoniumsalze, Alkanolammoniumsalze mit 2 bis 4 C-Atomen in der Alkanolgruppe wie z. B. das Monoethanolammoniumsalz, das Triethanolammoniumsalz oder das Isopropanolammoniumsalz zu verstehen. Die erfindungsgemäßen Haarfärbemittel können die direktziehenden Nitrodiphenylaminderivate der allgemeinen Formel I allein oder in Kombination mit bekannten direktziehenden Farbstoffen, z. B. mit anderen Nitrobenzolderivaten, Anthrachinonfarbstoffen, Triphenylmethan- oder Azofarbstoffen enthalten. Darüber hinaus eignen sich die direktziehenden Farbstoffe der allgemeinen Formel I wegen ihrer hohen Beständigkeit gegenüber Reduktionsmitteln und Oxidationsmitteln auch sehr gut zur Kombination mit Oxidationsfarbstoffvorprodukten, also zur Modifikation der Nuancen von Oxidationshaarfärbemitteln. Oxidationshaarfärbemittel enthalten als Farbstoffvorprodukte Entwicklerkomponenten, die durch oxidative Kupplung untereinander oder mit geeigneten Kupplerkomponenten die Oxidationsfarbstoffe ausbilden. Als Entwicklersubstanzen werden z. B. primäre aromatische Amine mit einer weiteren, in Para- oder Orthoposition befindlichen freien oder substituierten Hydroxy- oder Aminogruppe, Diaminopyridinderivate, heterocyclische Hydrazone, 4-Aminopyrazolonderivate und 2,4,5,6-Tetraaminopyrimidin und dessen Derivate eingesetzt. Als Kupplersubstanzen werden m-Phenylendiaminderivate, Naphthole, Resorcin und Resorcinderivate, Pyrazolone und m-Aminophenole verwendet.

Zur Herstellung der erfindungsgemäßen Haarfärbemittel werden die direktziehenden Haarfarbstoffe und gegebenenfalls die Oxidationsfarbstoffvorprodukte in einen geeigneten kosmetischen Träger, z. B. in Cremes, Emulsionen, Gele oder auch tensidhaltige, schäumende Lösungen, z. B. in Shampoos, Schaumaerosole oder andere Zubereitungen eingearbeitet, die für die Anwendung auf dem Haar geeignet sind.

Übliche Bestandteile solcher kosmetischer Zubereitungen sind z. B. Netz- und Emulgiermittel wie anionische, nichtionische oder ampholytische Tenside, z. B. Fettalkoholsulfate, Alkansulfonate, alpha-Olefinsulfonate, Fettalkoholpolyglykolethersulfate, Ethylenoxidanlagerungsprodukte an Fettalkohole, Fettsäuren und Alkylphenole, Sorbitanfettsäureester und Fettsäurepartialglyceride, Fettsäurealkanolamide sowie Verdickungsmittel wie z. B. Methyl- oder Hydroxyethylcellulose, Stärke, Fettalkohole, Paraffinöle, Fettsäuren, ferner Parfümöle und haarpflegende Zusätze, wie z. B. wasserlösliche kationische Polymere, Proteinderivate, Pantothensäure und Cholesterin. Die Bestandteile der kosmetischen Träger werden zur Herstellung der erfindungsgemäßen Haarfärbemittel in für diese Zwecke üblichen Mengen eingesetzt; z. B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

In den erfindungsgemäßen Haarfärbemitteln werden die direktziehenden Haarfarbstoffe der allgemeinen Formel I in einer Menge von 0,01 bis 5,0 Gew.-%, bevorzugt von 0,1 bis 2 Gew.-%, bezogen auf das

gesamte Haarfärbemittel, eingesetzt. Daneben können bekannte Oxidationshaarfärbemittelvorprodukte (Entwickler- und Kupplersubstanzen) in einer Menge von 0,01 bis 5, vorzugsweise von 1 bis 3 Gew.-% enthalten sein.

Wenn das erfindungsgemäße Haarfärbemittel Oxidationsfarbstoffvorprodukte enthält, empfiehlt sich außerdem die Zugabe einer kleinen Menge eines Reduktionsmittels, z. B. von 0,5 bis 2,0 Gew.-% Natriumsulfit zur Stabilisierung der Oxidationsfarbstoffvorprodukte. In diesem Falle wird vor der Anwendung des Haarfärbemittels dieses mit einem Oxidationsmittel versetzt, um die oxidative Entwicklung der Oxidationsfarbstoffvorprodukte einzuleiten. Als Oxidationsmittel kommen insbesondere Wasserstoffperoxid oder dessen Anlagerungsprodukte an Harnstoff, Melamin oder Natriumborat sowie Gemische aus derartigen Wasserstoffperoxidanlagerungsprodukten mit Kaliumperoxidsulfat in Frage.

Die Anwendung der erfindungsgemäßen Haarfärbemittel kann, unabhängig von der Art der kosmetischen Zubereitung, zum Beispiel als Creme, Gel oder Shampoo, im schwach sauren, neutralen oder alkalischen Milieu erfolgen. Bevorzugt ist die Anwendung der Haarfärbemittel in einem pH-Bereich von 8 bis 10. Die Anwendungstemperaturen können in einem Bereich zwischen 15 °C und 40 °C liegen. Nach einer Einwirkungszeit von ca. 30 Minuten wird das Haarfärbemittel durch Ausspülen von dem zu färbenden Haar entfernt. Danach wird das Haar mit einem milden Shampoo nachgewaschen und getrocknet. Das Nachwaschen mit einem Shampoo entfällt, wenn ein stark tensidhaltiger Träger, z. B. ein Färbeshampoo, verwendet wurde.

Mit den erfindungsgemäßen Haarfärbemitteln lassen sich Haaranfärbungen von hoher Intensität und guten Echtheitseigenschaften, besonders auch von guter Waschechtheit und hoher Stabilität gegen Ausbluten und Farbveränderungen beim Shamponieren erzielen. Die nachfolgenden Beispiele sollen den Erfindungsgegenstand näher erläutern, ohne ihn jedoch hierauf zu beschränken.

## Beispiele

Herstellungsbeispiele

1. 2-Amino-4-nitro-2'-methyl-diphenylamin-5'-carbonsäure

Stufe 1:

2,4-Dinitro-2'-methyl-diphenylamin-5'-carbonsäure-Na-Salz

Zu einer Mischung aus 7,7 g 3-Amino-4-methylbenzoesäure und 6 g Natriumcarbonat in einem Ethanol-Wasser-Gemisch (1 : 1 Vol.-Teile) wurden 9,4 g 2,4-Dinitrofluorbenzol zugetropft. Das Reaktionsgemisch wurde auf 70 °C erwärmt und nach 1 Stunde Reaktionszeit bei 70 °C auf 20 °C abgekühlt. Der gebildete Niederschlag wurde abfiltriert und bei 60 °C im Vakuumtrockenschrank getrocknet. Es wurden orange Kristalle mit einem Schmelzpunkt von 187 °C erhalten.

Stufe 2:

Zu einer Mischung aus 7,5 g des Produktes aus Stufe 1 und 0,8 g Natriumhydroxid in 300 ml eines Ethanol-Wasser-Gemisches (1 : 2 Vol.-Teile) wurde eine Mischung aus 2,75 g $Na_2S.3H_2O$und 1,34 g Schwefel in einem Gemisch aus 2 ml Wasser und 0,7 ml Ethanol zugegeben. Das Reaktionsgemisch wurde zum Sieden unter Rückfluß erhitzt, und nach einer Reaktionszeit von 3 Stunden wurde auf 20 °C abgekühlt, vom Ungelösten abfiltriert und vom Filtrat ca. 60 ml Lösungsmittel abdestilliert. Dann wurde das Filtrat durch Zugabe von konzentrierter Salzsäure neutralisiert (bis pH = 7) und der gebildete Niederschlag abfiltriert und aus Ethanol umkristallisiert. Es wurde ein olivfarbenes Pulver mit einem Schmelzpunkt von 229 bis 238 °C erhalten.

Die folgenden Beispiele 2 bis 7 wurden analog Beispiel 1 hergestellt:

2. 2-Amino-4-nitro-2'-methoxy-diphenylamin-5'-carbonsäure

Ausgangsprodukt: 3-Amino-4-methoxybenzoesäure

Stufe 1:

2,4-Dinitro-2'-methoxy-diphenylamin-5'-carbonsäure-Na-Salz Orangerote Kristalle, Schmelzpunkt: oberhalb 250 °C

Stufe 2:

Oranges Pulver, Schmelzpunkt 242 °C

3. 2-Amino-4-nitro-4'-methyl-diphenylamin-2'-carbonsäure

Ausgangsprodukt: 2-Amino-5-methyl-benzoesäure

Stufe 1:

2,4-Dinitro-4'-methyl-diphenylamin-2'-carbonsäure-Na-Salz Rotes Pulver, Schmelzpunkt oberhalb 250 °C

Stufe 2:
Braunes Pulver, Schmelzpunkt oberhalb 250 °C
4. 2-Amino-4-nitro-5'-chlor-diphenylamin-2'-carbonsäure
Ausgangsprodukt: 2-Amino-4-chlor-benzoesäure
Stufe 1:
2,4-Dinitro-5'-chlor-diphenylamin-2'-carbonsäure-Na-Salz Rotes Pulver, Schmelzpunkt oberhalb 250 °C
Stufe 2:
Grünes Pulver, Schmelzpunkt oberhalb 250 °C
5. 2-Amino-4-nitro-3'-methyl-diphenylamin-2'-carbonsäure
Ausgangsprodukt: 2-Amino-6-methyl-benzoesäure
Stufe 1:
2,4-Dinitro-3'-methyl-diphenylamin-2'-carbonsäure-Na-Salz Rotes Pulver, Schmelzpunkt oberhalb 250 °C
Stufe 2:
Rote Kristalle
IR-Spektrum (KBr, $cm^{-1}$): 1610, 1580, 1540, 1480, 1465, 1435, 1395, 1370, 1300, 1225, 1150, 1110, 1080, 960, 850, 810
6. 2-Amino-4-nitro-4'-methyl-diphenylamin-3'-sulfonsäure
Ausgangsprodukt: 6-Amino-m-toluolsulfonsäure
Stufe 1:
2,4-Dinitro-4'-methly-diphenylamin-2'-sulfonsäure-Na-Salz Orange Kristalle, Schmelzpunkt oberhalb 250 °C
Stufe 2:
Dunkelrote Kristalle, Schmelzpunkt oberhalb 250 °C .
7. 2-Amino-4-nitro-diphenylamin-3'-sulfonsäure
Ausgangssubstanz: m-Sulfanilsäure
Stufe 1:
2,4-Dinitro-diphenylamin-3'-sulfonsäure-Na-Salz Orange Kristalle, Schmelzpunkt 195 bis 207 °C (unter Zersetzung)
Stufe 2:
Olivgrünes Pulver, Schmelzpunkt 189 bis 194 °C

Folgende literaturbekannte Verbindungen wurden zusätzlich in die Haarfärbeversuche einbezogen:
8. 2-Amino-4-nitro-diphenylamin-4'-carbonsäure
(J. Prakt. Chem. (2) 91, 205)
9. 2-Amino-4-nitro-diphenylamin-3'-carbonsäure
(J. Prakt. Chem. (2) 91, 210)

## Haarfärbeversuche

Es wurden Haarfärbecremes der folgenden Zusammensetzung hergestellt:

| | |
|---|---|
| Fettalkohol $C_{12-18}$ | 10 g |
| Fettalkohol $C_{12-14}$ + 2 EO-sulfat, Na-Salz (28%ig) | 25 g |
| Wasser | 60 g |
| direktziehender Farbstoff | 1 g |
| Ammoniumsulfat | 1 g |
| konzentrierte Ammoniak-Lösung | bis pH = 9,5 |
| Wasser | ad 100 g |

Die Bestandteile wurden der Reihe nach miteinander vermischt. Nach Zugabe der direktziehenden Farbstoffe wurde mit konzentrierter Ammoniak-Lösung der pH-Wert der Emulsion auf 9,5 eingestellt, dann wurde mit Wasser auf 100 g aufgefüllt.

Die Färbecreme wurde auf ca. 5 cm lange Strähnen standardisierten, zu 90 % ergrauten, aber nicht besonders vorbehandelten Menschenhaars aufgetragen und dort 30 Minuten bei 27 °C belassen. Nach Beendigung des Färbeprozesses wurde das Haar gespült, mit einem üblichen Haarwaschmittel ausgewaschen und anschließend getrocknet.

Als direktziehende Haarfarbstoffe wurden die Verbindungen gemäß Beispiel 1 bis 9 eingesetzt.

Das Ergebnis der Färbeversuche ist der Tabelle I zu entnehmen.

## Tabelle I

| direktziehender Farbstoff gemäß Beispiel | Nuance des gefärbten Haares |
|---|---|
| 1 | strohgelb |
| 2 | mandarinengelb |
| 3 | topasgelb |
| 4 | aschblond |
| 5 | weizengold |
| 6 | hellorange |
| 7 | orange |
| 8 | buttergelb |
| 9 | mandarinenorange |

## Ansprüche

1. Die Verwendung von Verbindungen der Formel 1

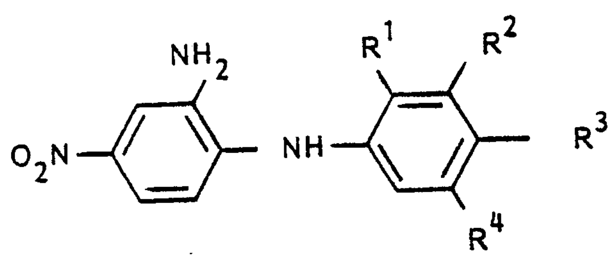

(I)

in der eine der Gruppen $R^1$ bis $R^4$ eine $-SO_3H$- oder eine -COOH-Gruppe ist und die drei anderen unabhängig voneinander Wasserstoff oder Alkylgruppen mit 1 bis 4 C-Atomen sind oder eine der anderen eine Alkoxygruppe mit 1 bis 4 C-Atomen oder Chlor und zwei Wasserstoff oder Alkylgruppen mit 1 bis 4 C-Atomen sind, und deren wasserlöslichen Salzen als direktziehende Haarfarbstoffe zum Färben von Haaren.

2. Die Verwendung nach Anspruch 1, dadurch gekennzeichnet, daß eine der Gruppen $R^1$ bis $R^4$ eine $SO_3H$ oder eine -COOH-Gruppe ist und die anderen Wasserstoff sind oder eine der anderen eine Methylgruppe, eine Methoxygruppe oder Chlor ist und zwei Wasserstoff sind.

3. Haarfärbemittel mit einem Gehalt an direktziehenden Haarfarbstoffen in einem kosmetischen Träger, dadurch gekennzeichnet, daß als direktziehende Haarfarbstoffe Verbindungen der Formel I gemäß Anspruch 1 oder 2 enthalten sind und diese gegebenenfalls gemeinsam mit weiteren direktziehenden Haarfarbstoffen und/oder Oxidationsfarbstoffvorproduktenin einem kosmetischen Träger in Form einer Creme, einer Emulsion, eines Gels, eines Shampoos oder eines Schaumaerosols eingearbeitet sind.

4. Haarfärbemittel nach den Ansprüchen 2 oder 3, dadurch gekennzeichnet, daß die direktziehenden Haarfarbstoffe der Formel I in einer Menge von 0,01 bis 5 Gew.-%, bezogen auf das gesamte Haarfärbemittel, enthalten sind.

5. Verbindungen der Formel I gemäß Anspruch 1, dadurch gekennzeichnet, daß eine der Gruppen $R^1$ bis $R^4$ eine $SO_3H$-Gruppe ist und zwei Wasserstoff sind, und deren wasserlösliche Salze.

6. Verbindungen der Formel I gemäß Anspruch 1, dadurch gekennzeichnet, daß eine der Gruppen $R^1$ bis $R^4$ eine -COOH-Gruppe, eine zweite eine Methylgruppe, eine Methoxygruppe oder Chlor ist und zwei Wasserstoff sind, und deren wasserlösliche Salze.

## Claims

1. The use of compounds corresponding to formula I

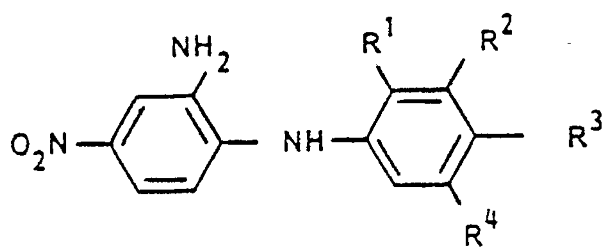

(I)

in which one of the substituents $R^1$ to $R^4$ is an $-SO_3H$ or a -COOH group and the other three independently of one another are hydrogen or $C_{1-4}$ alkyl groups or one of the others is a $C_{1-4}$ alkoxy group or chlorine and two are hydrogen or $C_{1-4}$ alkyl groups,
and water-soluble salts thereof as substantive hair dyes for dyeing hair.

2. The use claimed in claim 1, characterized in that one of the substituents $R^1$ to $R^4$ is an $-SO_3H$ or a -COOH group and the others are hydrogen or one of the others is a methyl group, a methoxy group or chlorine and two are hydrogen.

3. Hair-dyeing preparations containing substantive hair dyes in a cosmetic carrier, characterized in that they contain compounds corresponding to formula (I) as claimed in claim 1 or 2 as substantive dyes and these compounds are incorporated, optionally together with other substantive hair dyes and/or oxidation dye precursors, in a cosmetic carrier in the form of a cream, an emulsion, a gel, a shampoo or a foam aerosol.

4. Hair-dyeing preparations as claimed in claim 2 or 3, characterized in that the substantive hair dyes corresponding to formula (I) are present in a quantity of 0.01 to 5% by weight, based on the hair-dyeing preparation as a whole.

5. Compounds corresponding to formula I in claim 1, characterized in that one of the substituents $R^1$ to $R^4$ is an $SO_3H$ group and two are hydrogen, and water-soluble salts thereof.

6. Compounds corresponding to formula I in claim 1, characterized in that one of the groups $R^1$ to $R^4$ is a -COOH group, a second is a methyl group, a methoxy group or chlorine and two are hydrogen, and water-soluble salts thereof.

## Revendications

1. Utilisation comme colorants montant directement sur la fibrè pour la coloration des cheveux de composés de formule I

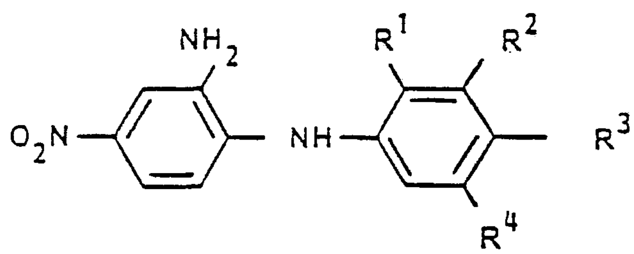

(I)

dans laquelle un des groupements $R^1$ à $R^4$ est un groupe $-SO_3H$ ou un groupe -COOH et les trois autres représentent indépendamment l'un de l'autre l'hydrogène ou des groupements alkyle comportant 1 à 4 atomes de C, ou bien un des autres représente un groupe alcoxy comportant 1 à 4 atomes de C ou le chlore, les deux autres qui restent étant constitués par de l'hydrogène ou des groupes alkyle comportant 1 à 4 atomes de C, et leurs sels solubles dans l'eau.

2. Utilisation selon la revendication 1, caractérisée en ce que l'un des groupements $R^1$ à $R^4$ est un groupe $-SO_3H$ ou un groupe -COOH et les autres sont l'hydrogène ou l'un des autres est un groupe méthyle, un groupe méthoxy ou le chlore, les deux derniers étant l'hydrogène.

3. Teintures pour cheveux contenant des colorants pour cheveux montant directement sur la fibre ou directs dans un support cosmétique, caractérisées en ce que sont contenus comme colorants pour cheveux directs des composés de la formule I, conformément à la revendication 1 ou 2, et en ce que ceux-ci sont incorporés le cas échéant conjointement avec d'autres colorants directs et/ou avec des précurseurs de colorants d'oxydation dans un support cosmétique sous forme de crème, d'émulsion, de gel, de shampooing ou d'aérosol de mousse.

4. Teintures pour cheveux selon la revendication 2 ou 3, caractérisées en ce que les colorants directs de la formule I sont contenus dans une proportion allant de 0,01 à 5 % en poids par rapport à la totalité de la teinture pour cheveux.

5. Composés de la formule I, conformément à la revendication 1, caractérisés en ce que l'un des groupements $R^1$ à $R^4$ est un groupe $-SO_3H$ et deux groupes sont l'hydrogène, et leurs sels solubles dans l'eau.

6. Composés de la formule I, conformément à la revendication 1, caractérisés en ce que l'un des groupements $R^1$ à $R^4$ est un groupe -COOH, un deuxième est un groupe méthyle, un groupe méthoxy ou le chlore, les deux derniers étant l'hydrogène, et leurs sels solubles dans l'eau.